(19)

Europäisches Patentamt

European Patent Office

Office européen des brevets

(11) **EP 3 644 092 A1**

(12) **EUROPEAN PATENT APPLICATION**

(43) Date of publication:
**29.04.2020 Bulletin 2020/18**

(51) Int Cl.:
**G01S 15/89** (2006.01)        **G01S 7/52** (2006.01)

(21) Application number: **18202321.8**

(22) Date of filing: **24.10.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **IMEC vzw**
**3001 Leuven (BE)**

(72) Inventors:
• **DEMI, Libertario**
  **3001 Leuven (BE)**
• **ROCHUS, Veronique**
  **3001 Leuven (BE)**

(74) Representative: **DenK iP**
  **Hundelgemsesteenweg 1116**
  **9820 Merelbeke (BE)**

(54) **ULTRASOUND IMAGING USING A NULL SUBTRACTION IMAGING TECHNIQUE**

(57)    In a first aspect, the present invention relates to a method for visualizing an interface between an epidermis and an external environment. The method comprises:

(a) acquiring an ultrasound signal of the interface using an ultrasonic transducer array; and (b) applying a null subtraction imaging technique to the acquired ultrasound signal, thereby forming an ultrasound image.

**FIG. 1**

**Description**

**Technical field of the invention**

The present invention relates to ultrasound imaging of an interface between an epidermis and an external environment and particularly to signal processing techniques used for said ultrasound imaging.

**Background of the invention**

Fingerprint imaging requires a high resolution (e.g. 100 $\mu$m or better) in order to visualize fingerprint features with sufficient accuracy to guarantee a reliable recognition. Ultrasound imaging is a very interesting and promising technology for fingerprint imaging, offering a robust imaging (e.g. being less susceptive than capacitive sensors to contamination and moisture on the finger) and enabling a high level of security. An easy, cheap, robust and reliable fingerprint imaging is for example useful in view of securing the access to an increasing number of items, such personal electronic devices (e.g. phone, tablet, watch, laptop or PC), personal data (e.g. bank, work, social security and health data), doors, luggage, etc.

However, using signal processing techniques which are commonly used up to now (e.g. standard linear beamforming), a high ultrasound frequency must be used to achieve a high spatial resolution. Indeed, the fundamental physical limit for resolution is normally dictated by diffraction. For a rectangular aperture with lateral size L, the theoretical -12 dB lateral spatial resolution $L_r$ can

$$1.6\frac{cz}{Lf_0},$$

be evaluated using the formula $L_r =$ wherein c is the speed of sound, z is the depth and $f_0$ is the center frequency of the transmitted pulse (c.f. Demi, Libertario, et al. "Implementation of parallel transmit beamforming using orthogonal frequency division multiplexing-achievable resolution and interbeam interference." IEEE transactions on ultrasonics, ferroelectrics, and frequency control 60.11 (2013): 2310-2320). As such, to achieve a resolution of at least 100 $\mu$m, frequencies of about 20 MHz or higher are required for a speed of sound of 5600 m/s (e.g. glass), an imaging depth of 1 mm and a lateral aperture size of 5 mm. This puts stringent requirements on the transmitting and receiving electronics, and on the aperture size (e.g. on the total number of ultrasonic transducer elements in an ultrasonic transducer array). Additionally, standard beamforming requires the ability to accurately control the phase of the signals used to drive each transducer element, with an accuracy in the order or higher than a tenth of the wavelength. However, this is increasingly difficult to achieve with increasing driving frequencies. Moreover, since attenuation of a sound wave is a frequency dependent phenomenon, where attenuation increases with frequency, using high frequencies has a negative impact on the propagation of the ultrasound wave. The signal-to-noise ratio and achievable imaging depths therefore deteriorate for higher frequencies.

There is therefore still a need in the art for better methods for ultrasound imaging, e.g. for fingerprint imaging, particularly ones which would allow for lower ultrasound frequencies to be used for a given aperture size (e.g. the total number of ultrasonic transducer elements in an ultrasonic transducer array) while maintaining a high resolution.

**Summary of the invention**

It is an object of the present invention to provide good methods and associated products for visualizing an interface between an epidermis and an external environment. This objective is accomplished by methods, products and a use according to the present invention.

In a first aspect, the present invention relates to a method for visualizing an interface between an epidermis and an external environment. The method comprises:

(a) acquiring an ultrasound signal of the interface using an ultrasonic transducer array;
and (b) applying a null subtraction imaging technique to the acquired ultrasound signal, thereby forming an ultrasound image.

It is an advantage of embodiments of the present invention that a high-resolution visualization can still be achieved at relatively low ultrasound frequencies. Keeping other factors the same, an improved in the resolution by a factor 5 or more, such as a factor of 8 or 20 or more, compared to standard linear beamforming could for instance be achieved. It is a further advantage of embodiments of the present invention that a super-resolution visualization can be made, i.e. that a resolution going beyond the boundary set by the traditional diffraction limit can be achieved.

It is an advantage of embodiments of the present invention that, for a given resolution, comparatively lower ultrasound frequencies can be used than those used in the methods of the known art (e.g. standard linear beamforming). It is a further advantage of the present invention that the impact of attenuation can be reduced by the use of lower ultrasound frequencies, leading to an improved signal-to-noise ratio and an improved image depth.

It is an advantage of embodiments of the present invention that the visualization of the interface can be achieved with high contrast.

It is an advantage of embodiments of the present invention that the method is robust against contamination and/or moisture on the interface.

It is an advantage of embodiments of the present invention that there may be no need, or a reduced need, for controlling the phase of the ultrasound hardware driving signals (e.g. for performing plane wave compounding with different steering angles) and for focusing of either or both of the emitted ultrasound wave and the received ultrasound echo, with the aim to further improve spatial resolution.

In embodiments, acquiring an ultrasound signal of the interface may comprise the substeps of: (a1) emitting an ultrasonic wave towards the interface; and (a2) receiving an ultrasound echo from the interface, thereby obtaining the ultrasound signal.

In embodiments, applying the null subtraction imaging technique may comprise the substeps of: (b1) applying a first apodization weight to the acquired ultrasound signal, thereby generating a first apodized signal; (b2) applying a second apodization weight to the acquired ultrasound signal, thereby generating a second apodized signal; (b3) applying a third apodization weight to the acquired ultrasound signal, thereby generating a third apodized signal; and (b4) combining the first, second and third apodized signals to form the ultrasound image.

It is an advantage of embodiments of the present invention that the null subtraction imaging technique may be based on three or more apodized signals.

In embodiments, the ultrasonic transducer array may comprise a transducer array aperture, and the first apodization weight may have a zero mean value across the transducer array aperture and the second and third apodization weights may have a non-zero mean value across the transducer array aperture.

In embodiments, the second apodization weight may be obtained by adding a DC bias to the first apodization weight and the third apodization weight may be a transpose of the second apodization weight.

In embodiments, combining the first, second and third apodized signals may comprise making a linear combination of the first, second and third apodized signals.

It is an advantage of embodiments of the present invention that the method may be performed in a relatively straightforward and economic fashion.

In a second aspect, the present invention relates to a method for scanning a fingerprint. The method comprises the method for visualizing an interface (e.g. of a finger) between an epidermis and an external environment according to an embodiment of the first aspect and further comprises a step c of: (c) processing the ultrasound image to obtain data indicative of the fingerprint.

It is an advantage of embodiments of the present invention that a fingerprint can be scanned and that corresponding indicative fingerprint data can be generated, e.g. for storage of the scanned fingerprint and/or for comparison to other fingerprint data.

In a third aspect, the present invention relates to a device comprising means for carrying out the steps of the method according to an embodiment of the first or second aspect.

It is an advantage of embodiments of the present invention that the requirements on the transmitting and receiving hardware (e.g. receiver, transmitter and/or associated elements, such as electronics) and on the aperture size (e.g. the size of the transducer array), can be relaxed; for example, because the ultrasound frequency can be relatively low and/or because there may be no need for phase control and/or focusing. It is a further advantage of embodiments of the present invention that this can result in devices of smaller dimensions, which is for example useful in mobile applications (e.g. mobile phones) where the available space is highly limited.

It is an advantage of embodiments of the present invention that, provided a corresponding adaptation of their ultrasound signal processing is foreseen, at least some of the presently known ultrasound imaging devices (e.g. at least some of the known ultrasound fingerprint scanners) could be used for performing the method. It is a further advantage of embodiments of the present invention that such an adaption could in some systems be substantially achieved through a software implementation, e.g. without necessarily requiring a complete redesign of the hardware (e.g. particularly the ultrasound hardware).

In embodiments, the device may comprise an ultrasound detector.

In embodiments, the device may comprise an ultrasound emitter.

It is an advantage of embodiments of the present invention that either a distinct ultrasonic receiver and an ultrasonic transmitter can be used, or that a combined ultrasonic transceiver can be used.

In embodiments, the device may be a fingerprint scanner.

In a fourth aspect, the present invention relates to a computer program product comprising instructions which, when the program is executed on a computer, cause the computer to carry out the steps of the method according to an embodiment of the first or second aspect.

In a fifth aspect, the present invention relates to a computer-readable medium comprising instructions which, when executed by a computer, cause the computer to carry out the steps of the method according to an embodiment of the first or second aspect.

In a sixth aspect, the present invention relates to a use of ultrasound imaging for visualizing an interface between an epidermis and an external environment, wherein the ultrasound imaging uses a null subtraction imaging technique.

In embodiments, the use may be for visualizing a fingerprint.

Particular and preferred aspects of the invention are set out in the accompanying independent and dependent claims. Features from the dependent claims may be combined with features of the independent claims and with features of other dependent claims as appropriate and not merely as explicitly set out in the claims.

Although there has been constant improvement, change and evolution of devices in this field, the present concepts are believed to represent substantial new and novel improvements, including departures from prior practices, resulting in the provision of more efficient, stable and reliable devices of this nature.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

## Brief description of the drawings

FIG. 1 is a flow-chart in accordance with embodiments of the present in invention.
FIG. 2 shows experimentally obtained acoustic lateral resolutions in function of the number of elements used in an ultrasonic transducer array, for different processing techniques and processing parameters in accordance with the prior art and with embodiments of the present invention.

In the different figures, the same reference signs refer to the same or analogous elements.

## Description of illustrative embodiments

The present invention will be described with respect to particular embodiments and with reference to certain drawings, but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

The terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, under and the like in the description and the claims are used for descriptive purposes and not necessarily for describing relative positions. It is to be understood that the terms so used are interchangeable with their antonyms under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Similarly, it is to be noticed that the term "coupled", also used in the claims, should not be interpreted as being restricted to direct connections only. The terms "coupled" and "connected", along with their derivatives, may be used. It should be understood that these terms are not intended as synonyms for each other. Thus, the scope of the expression "a device A coupled to a device B" should not be limited to devices or systems wherein an output of device A is directly connected to an input of device B. It means that there exists a path between an output of A and an input of B which may be a path including other devices or means. "Coupled" may mean that two or more elements are either in direct physical or electrical contact, or that two or more elements are not in direct contact with each other but yet still co-operate or interact with each other.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practised without these specific details. In other instances, well-known methods, structures and techniques have not been shown in detail in order not to obscure an understanding of this description.

The following terms are provided solely to aid in the understanding of the invention.

As used herein, and unless otherwise specified, an epidermis is the outermost layer of skin of a body part of a human or another animal (e.g. a vertebrate animal), or the outermost skin cell layer of a plant part.

As used herein, and unless otherwise specified, an ultrasonic transducer array is an array of a plurality of ultrasonic transducer elements. In embodiments, the ultrasonic transducer may be an ultrasonic receiver, an ultrasonic transmitter or an ultrasonic transceiver.

In a first aspect, the present invention relates to a method for visualizing an interface between an epidermis and an external environment. The method comprises:

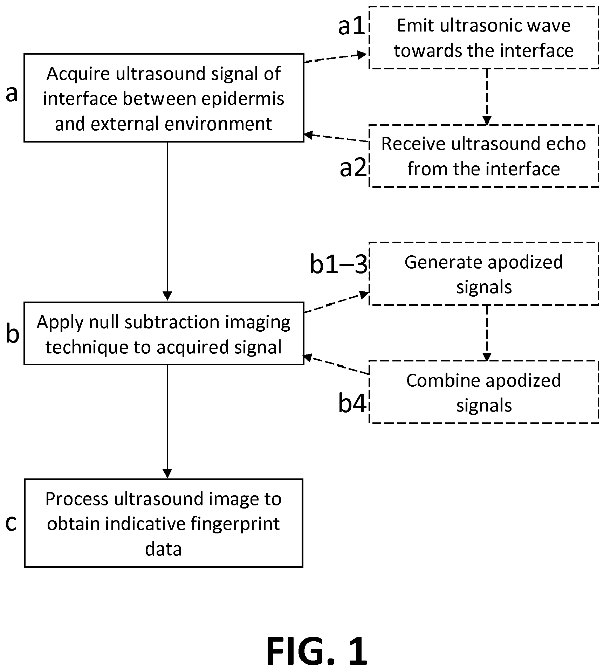

(a) acquiring an ultrasound signal of the interface using an ultrasonic transducer array;
and (b) applying a null subtraction imaging technique to the acquired ultrasound signal, thereby forming an ultrasound image. These steps are schematically depicted as steps a and b in the flow-chart of FIG. 1.

In embodiments, the epidermis may be an epidermis of a human or animal body part. In preferred embodiments, the body part may be a finger or a portion of a finger (e.g. a top portion of a finger, such as a fingertip). In embodiments, the interface that is visualized may correspond to a fingerprint.

In embodiments, the external environment may comprise (e.g. consist of) a fluid. In embodiments, the fluid may be a gas (e.g. air, such as ambient air) and/or a liquid (e.g. a liquid with a high acoustical impedance mismatch with respect to the epidermis). For example, the epidermis may be in contact with the gas, or may be submerged in the liquid, or may have a layer or droplets of the liquid thereon (e.g. as in the case of a wet or moist body part, such as a wet or moist finger).

In embodiments, the ultrasonic transducer array may comprise 2 or more ultrasonic transducer elements, preferably from 2 to 20, yet more preferably from 4 to 12, most preferably from 6 to 10, such as 8 or more ultrasonic transducer elements. In embodiments, the ultrasonic transducer array (e.g. ultrasonic emitter and/or receiver) may operate at an ultrasound frequency of from 1 to 50 MHz, preferably from 5 to 20 MHz, such as from 10 to 15 Mhz.

In embodiments, acquiring an ultrasound signal of the interface may comprise the substeps of: (a1) emitting an ultrasonic wave towards the interface; and (a2) receiving an ultrasound echo from the interface, thereby obtaining the ultrasound signal. These steps are schematically depicted as steps a1 and a2 in the flow-chart of FIG. 1. In embodiments, emitting an ultrasonic wave may comprise using an ultrasound emitter (e.g. an ultrasound transmitter or an ultrasound transceiver). In embodiments, receiving the ultrasound echo may comprise using an ultrasound detector (e.g. an ultrasound receiver or an ultrasound transceiver). In embodiments, the ultrasound emitter may be a first ultrasonic transducer array and/or the ultrasound receiver may be a second ultrasonic transducer array. In embodiments, the first ultrasonic transducer array and the second ultrasonic transducer array may be a same ultrasonic transducer array or may be two different ultrasonic transducer arrays. In embodiments in which the first ultrasonic transducer array and the second ultrasonic transducer array are the same ultrasonic transducer array, said array may be the ultrasonic transducer array referred to in step (a). In embodiments in which the first ultrasonic transducer array and the second ultrasonic transducer array are different ultrasonic transducer arrays, either of the first ultrasonic transducer array or the second ultrasonic transducer array may be the ultrasonic transducer array referred to in step a.

The use of the null subtraction imaging technique is known in the field of (bio)medical ultrasound imaging, for example from Reeg (2016) (Reeg, Jonathan R. Null subtraction imaging technique for biomedical ultrasound imaging. 2016. PhD Thesis). The field of (bio)medical ultrasound imaging deals with ultrasound imaging of the interior of a human or animal body, aiming to visualize internal tissues, nerves, veins, muscles, tumours, cellular structures, etc. However, a disadvantage of null subtraction imaging signal processing technique is that, while the resolution of the constructed image is improved, the contrast thereof decreases. This trade-off is not always worthwhile and, consequently, Reeg (2016) states: "For contrast targets, a decrease in CNR [i.e. contrast-to-noise ratio] was observed that could not fully be compensated for. This makes our imaging technique ideal for the detection of small, highly reflective targets, but not a one-size-fits-all approach for all ultrasound imaging tasks." (Reeg, 2016, p. 35).

However, it was surprisingly realized within the present invention that imaging of the epidermis for e.g. fingerprint scanning is in fact not an imaging of the epidermal tissue as such, but a visualization of the interface between the epidermis and the outside environment. This leads to a number of considerably distinct conditions for this type of imaging, which are not typically found in (bio)medical ultrasound imaging; thereby setting itself apart from that field. First, whereas different tissues and structures inside the body are typically characterized by relatively small acoustic impedance differences, there is a high acoustical impedance mismatch between the epidermis and the outside environment (e.g. air). Second, whereas the interior of the body comprises a large number of different structures and tissues, the visualization of the epidermal interface corresponds to the visualization of a binary object: the epidermal tissue (e.g. ridges in a fingerprint) and the outside environment (e.g. valleys in a fingerprint that are filled with air). Third, whereas the interior of the body typically has structures ranging in size from several tens of cm to as low as a few nm and typically comprises a dense concentration of randomly oriented scatterers, the features (e.g. ridges and valleys) to be visualized of the epidermal interface have sizes which are compatible with the targeted spatial resolution (e.g. in the order of 100 $\mu$m) and typically have a more organized orientation. Fourth, the visualization of the epidermal interface may typically require only one (or two) imaging planes to be measured; furthermore, these imaging planes are typically at a fixed depth which is known a priori. As such, it was for the first time recognized that null subtraction imaging can be particularly advantageously used in the ultrasound imaging of the epidermal interface, thereby improving the resolution of the obtained image without being overly negatively impacted by the loss of contrast. In this context, it is worth noting that the epidermal interface is not a 'small, highly reflective target' and this is therefore indeed not an application which was anticipated by Reeg (2016).

In embodiments, applying the null subtraction imaging technique may comprise the substeps of: separately applying two or more apodization weights to the acquired ultrasound signal, thereby generating two or more apodized signals and subsequently combining the two or more apodized signals to form the ultrasound image. In embodiments, the ultrasonic transducer array may comprise a transducer array aperture, and at least one of the apodization weights may have a zero mean value across the transducer array aperture, while at least one other of the two or more apodization weights may have a non-zero mean value across the transducer array aperture. In embodiments, combining the two or more apodized signals may comprise making a linear combination of the two or more apodized signals.

In preferred embodiments, applying the null subtraction imaging technique may be performed by the 'bridging approach'; cf. Reeg (2016, p. 7-9, chapter 3.2), which is incorporated herein by reference. In embodiments, applying the null subtraction imaging technique may comprise the substeps of: (b1) applying a first apodization weight to the acquired ultrasound signal, thereby generating a first apodized signal; (b2) applying a second apodization weight to the acquired ultrasound signal, thereby generating a second apodized signal; (b3) applying a third apodization weight to the acquired ultrasound signal, thereby generating a third apodized signal; and (b4) combining the first, second and third apodized signals to form the ultrasound image. These steps are schematically depicted as steps b1-3 and b4 in the flowchart of FIG. 1. In embodiments, the ultrasonic transducer array may comprise a transducer array aperture, and the first apodization weight may have a zero mean value across the transducer array aperture and the second and third apodization weights may have a non-zero mean value across the transducer array aperture. In embodiments, the second apodization weight may be obtained by adding a DC bias to the first apodization weight and the third apodization weight may be a transpose of the second apodization weight. In embodiments, combining the first, second and third apodized signals may comprise making a linear combination of the first, second and third apodized signals. In embodiments, the linear combination may comprise subtracting the first apodized signal from a sum of the second and third apodized signals.

In other embodiments, applying the null subtraction imaging technique may be performed by the 'masking approach'; cf. Reeg (2016, p. 7, chapter 3.1), which is incorporated herein by reference. In embodiments, applying the null subtraction imaging technique may comprise the substeps of: (b1) applying a first apodization weight to the acquired ultrasound signal, thereby generating a first apodized signal; (b2) applying a second apodization weight to the acquired ultrasound signal, thereby generating a second apodized signal; and (b4) combining the first and second apodized signals to form the ultrasound image. In embodiments, the ultrasonic transducer array may comprise a transducer array aperture, and the first apodization weight may have a zero mean value across the transducer array aperture and the second apodization weights may have a non-zero mean value across the transducer array aperture. In embodiments, the two main lobes of the first apodized signal may be such that they mask the main lobe of the second apodized signal. In embodiments, the second apodization weight may correspond to a rectangular apodization. In embodiments, the first apodization weight may correspond to a single-square wave cycle apodization. In embodiments, combining the first and second apodized signals may comprise making a linear combination of the first and second apodized signals. In embodiments, the linear combination may comprise scaling the second apodized signal and subsequently subtracting the first apodized signal therefrom.

In yet other embodiments, applying the null subtraction imaging technique may be performed by yet another approach.

In embodiments, the method may further be for visualizing a layer beyond the interface (e.g. for visualizing the epidermis or the dermis). Visualizing beyond the interface can advantageously allow determining, for example, the pulsatility in the target (e.g. in the fingertip). This can allow a further level of security, e.g. in fingerprint scanning, because it can allow verifying that what is being imaged is indeed the epidermal interface of a living being and not a replica thereof. Because the method for visualizing the epidermal interface in accordance with embodiments of the present invention can operate at lower ultrasound frequencies, a lower attenuation and deeper penetration of the ultrasound waves can advantageously be achieved. If the loss of contrast by using null subtraction imaging for the deeper layer becomes too predominant, it can be considered to combine null subtraction imaging for the high-resolution visualization of the epidermal interface and standard linear beamforming (or another technique) for the higher contrast (but at the cost of a loss of resolution) visualization of the deeper layer.

In embodiments, any feature of the first aspect or its embodiments may independently be as correspondingly described for embodiments of any other aspect.

In a second aspect, the present invention relates to a method for scanning a fingerprint. The method comprises the method for visualizing an interface (e.g. of a finger) between an epidermis and an external environment according to an embodiment of the first aspect and further comprises a step c of: (c) processing the ultrasound image to obtain data indicative of the fingerprint (e.g. to obtain a digital fingerprint pattern, to obtain a string representative of the fingerprint, etc.). This step is schematically depicted as steps c in the flow-chart of FIG. 1.

In embodiments, the data indicative of the fingerprint may be suitable for comparing the scanned fingerprint to one or more known fingerprints, which may for example also be in the form of corresponding indicative fingerprint data. The known fingerprints data may, for example, have been obtained from a previous scan and/or may be retrieved from a database. Various algorithms are known in the art for processing an image of a fingerprint (e.g. the ultrasound image; cf. step c) on the one hand, and for comparing (which can also be referred to as 'matching') indicative fingerprint data on the other hand; as such, these are not further discussed here.

In embodiments, any feature of the second aspect or its embodiments may independently be as correspondingly described for embodiments of any other aspect.

In a third aspect, the present invention relates to a device comprising means for carrying out the steps of the method according to an embodiment of the first or second aspect.

In embodiments, the device may comprise an ultrasound detector (e.g. an ultrasound receiver or an ultrasound transceiver).

In embodiments, the device may comprise an ultrasound emitter (e.g. an ultrasound transmitter or an ultrasound transceiver).

In embodiments, the ultrasound detector and/or ultrasound emitter may be an ultrasonic transducer array.

In embodiments, the device may be a fingerprint scanner.

In embodiments, any feature of the third aspect or its embodiments may independently be as correspondingly described for embodiments of any other aspect.

In a fourth aspect, the present invention relates to a computer program product comprising instructions which, when the program is executed on a computer, cause the computer to carry out the steps of the method according to an embodiment of the first or second aspect.

In embodiments, any feature of the fourth aspect or its embodiments may independently be as correspondingly described for embodiments of any other aspect.

In a fifth aspect, the present invention relates to a computer-readable medium comprising instructions which, when executed by a computer, cause the computer to carry out the steps of the method according to an embodiment of the first or second aspect.

In embodiments, any feature of the fifth aspect or its embodiments may independently be as correspondingly described for embodiments of any other aspect.

In a sixth aspect, the present invention relates to a use of ultrasound imaging for visualizing an interface between an epidermis and an external environment, wherein the ultrasound imaging uses a null subtraction imaging technique.

In embodiments, the use may be for visualizing a fingerprint.

In embodiments, any feature of the sixth aspect or its embodiments may independently be as correspondingly described for embodiments of any other aspect.

The invention will now be described by a detailed description of several embodiments of the invention. It is clear that other embodiments of the invention can be configured according to the knowledge of the person skilled in the art without departing from the true technical teaching of the invention, the invention being limited only by the terms of the appended claims.

Example: visualizing a wire target in water

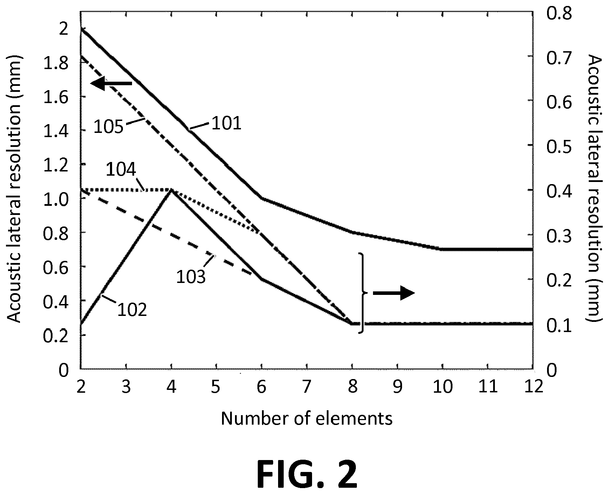

A linear ultrasonic transducer array having a pitch equal to 100 microns was employed as a transceiver to image a wire target in water, using a 15 MHz ultrasonic imaging frequency. A plane wave emission was used, without focusing, neither in transmit nor in receive mode. Ultrasound images were then generated, each time by processing the ultrasound signal acquired from a single transmission event. FIG. 2 shows the achieved -6 dB acoustic lateral resolution for different processing techniques and processing parameters, in function of the number of elements used in the ultrasonic transducer array.

Curve 101 corresponds to the processing of the ultrasound signal by standard linear beamforming, as is known in the prior art. As seen in FIG.2, a resolution of about 700 $\mu$m was achieved with standard beamforming using 10 or more elements. By comparison, a diffraction limited lower boundary of about 550 $\mu$m can be expected under these conditions.

Conversely, curves 102-105 correspond to the processing of the ultrasound signal by applying a 'bridging approach' null subtraction imaging, in accordance with embodiments of the present invention. The difference between curves 102-105 is in the value that was used for the DC bias in the generation of the apodized signals: 0.10 for 102, 0.15 for 103, 0.20 for 104 and 0.25 for 105. As seen in FIG.2, and regardless of the DC bias that was used, the achieved resolution is consistently better using null subtraction imaging than standard beamforming for a given number of elements. Furthermore, curves 102-105 converge for increasing elements, and the achieved resolution reaches a lower limit of about 100 $\mu$m for 8 elements and upwards; i.e. considerably below both the best practical optimum of 700 $\mu$m achieved with standard beamforming and the theoretical diffraction limited optimum of 550 $\mu$m. Super-resolution values are thus made accessible through null subtraction imaging. On top of that, the optimum was reached for 8 elements (compared to 10 for standard beamforming), indicating that furthermore fewer elements are needed when using null subtraction imaging.

The wire target in water in this example can be considered as a model case for the visualization of an interface between an epidermis and an external environment (e.g. for the visualization of a fingerprint). As such, similar results are expected for the visualization of such an interface.

It is to be understood that although preferred embodiments, specific constructions and configurations, as well as materials, have been discussed herein for devices according to the present invention, various changes or modifications in form and detail may be made without departing from the scope and technical teachings of this invention. For example, any formulas given above are merely representative of procedures that may be used. Functionality may be added or deleted from the block diagrams and operations may be interchanged among functional blocks. Steps may be added or deleted to methods described within the scope of the present invention.

## Claims

1. A method for visualizing an interface between an epidermis and an external environment, comprising:

   a. acquiring an ultrasound signal of the interface using an ultrasonic transducer array, and
   b. applying a null subtraction imaging technique to the acquired ultrasound signal, thereby forming an ultrasound image.

2. The method according to claim 1, wherein acquiring an ultrasound signal of the interface comprises the substeps of:

   a1. emitting an ultrasonic wave towards the interface, and
   a2. receiving an ultrasound echo from the interface, thereby obtaining the ultrasound signal.

3. The method according to any of the previous claims, wherein applying the null subtraction imaging technique comprises the substeps of:

   b1. applying a first apodization weight to the acquired ultrasound signal, thereby generating a first apodized signal,
   b2. applying a second apodization weight to the acquired ultrasound signal, thereby generating a second apodized signal,
   b3. applying a third apodization weight to the acquired ultrasound signal, thereby generating a third apodized signal, and
   b4. combining the first, second and third apodized signals to form the ultrasound image.

4. The method according to claim 3, wherein the ultrasonic transducer array comprises a transducer array aperture and wherein the first apodization weight has a zero mean value across the transducer array aperture and wherein the second and third apodization weights have a non-zero mean value across the transducer array aperture.

5. The method according to claim 4, wherein the second apodization weight is obtained by adding a DC bias to the first apodization weight and wherein the third apodization weight is a transpose of the second apodization weight.

6. The method according to any of claims 3 to 5, wherein combining the first, second and third apodized signals comprises making a linear combination of the first, second and third apodized signals.

7. A method for scanning a fingerprint, comprising the method for visualizing an interface between an epidermis and an external environment of a finger according to any of the previous claims and further comprising a step c of:

> c. processing the ultrasound image to obtain data indicative of the fingerprint.

8. A device comprising means for carrying out the steps of the method according to any of the previous claims.

9. The device according to claim 8, comprising an ultrasound detector.

10. The device according to claim 8 or 9, comprising an ultrasound emitter.

11. The device according to any of claims 8 to 10, being a fingerprint scanner.

12. A computer program product comprising instructions which, when the program is executed on a computer, cause the computer to carry out the steps of the method according to any of claims 1 to 7.

13. A computer-readable medium comprising instructions which, when executed by a computer, cause the computer to carry out the steps of the method according to any of claims 1 to 7.

14. Use of ultrasound imaging for visualizing an interface between an epidermis and an external environment, wherein the ultrasound imaging uses a null subtraction imaging technique.

15. The use according to claim 14, for visualizing a fingerprint.

a1 | Emit ultrasonic wave towards the interface

a | Acquire ultrasound signal of interface between epidermis and external environment

a2 | Receive ultrasound echo from the interface

b1–3 | Generate apodized signals

b | Apply null subtraction imaging technique to acquired signal

b4 | Combine apodized signals

c | Process ultrasound image to obtain indicative fingerprint data

# FIG. 1

**FIG. 2**

Europäisches
Patentamt

European
Patent Office

Office européen
des brevets

## EUROPEAN SEARCH REPORT

Application Number

EP 18 20 2321

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2017/090028 A1 (DJORDJEV KOSTADIN DIMITROV [US] ET AL) 30 March 2017 (2017-03-30) * abstract *; figures 1A-4,6A-7D,9-15B * * paragraph [0001] - paragraph [0018] * * paragraph [0047] - paragraph [0062] * * paragraph [0097] - paragraph [0124] * ----- | 1-15 | INV. G01S15/89 G01S7/52 |
| Y | US 2017/108584 A1 (OELZE MICHAEL L [US] ET AL) 20 April 2017 (2017-04-20) * the whole document * ----- | 1-15 | |
| Y | Jonathan R Reeg: "NULL SUBTRACTION IMAGING TECHNIQUE FOR BIOMEDICAL ULTRASOUND IMAGING", , 7 July 2016 (2016-07-07), pages 1-37, XP055476956, Retrieved from the Internet: URL:http://hdl.handle.net/2142/90529 [retrieved on 2018-05-18] * the whole document * ----- | 1-15 | |
| A | US 6 645 145 B1 (DRESCHEL WILLIAM R [US] ET AL) 11 November 2003 (2003-11-11) * abstract *; figures 3, 4C, 5 * * column 5, line 36 - line 59 * * column 11, line 64 - column 12, line 33 * * column 15, line 44 - line 53 * ----- | 1-15 | TECHNICAL FIELDS SEARCHED (IPC) G01S |

The present search report has been drawn up for all claims

| Place of search | Date of completion of the search | Examiner |
|---|---|---|
| Munich | 5 April 2019 | Zaneboni, Thomas |

EPO FORM 1503 03.82 (P04C01)

**ANNEX TO THE EUROPEAN SEARCH REPORT
ON EUROPEAN PATENT APPLICATION NO.**

EP 18 20 2321

05-04-2019

| Patent document cited in search report | | Publication date | Patent family member(s) | | Publication date |
|---|---|---|---|---|---|
| US 2017090028 | A1 | 30-03-2017 | BR 112018005977 | A2 | 23-10-2018 |
| | | | CA 2997085 | A1 | 30-03-2017 |
| | | | CN 108140106 | A | 08-06-2018 |
| | | | EP 3353708 | A1 | 01-08-2018 |
| | | | JP 2018535712 | A | 06-12-2018 |
| | | | KR 20180059504 | A | 04-06-2018 |
| | | | US 2017090028 | A1 | 30-03-2017 |
| | | | WO 2017052836 | A1 | 30-03-2017 |
| US 2017108584 | A1 | 20-04-2017 | NONE | | |
| US 6645145 | B1 | 11-11-2003 | NONE | | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DEMI, LIBERTARIO et al.** Implementation of parallel transmit beamforming using orthogonal frequency division multiplexing-achievable resolution and interbeam interference. *IEEE transactions on ultrasonics, ferroelectrics, and frequency control,* 2013, vol. 60 (11), 2310-2320

- **REEG, JONATHAN R.** Null subtraction imaging technique for biomedical ultrasound imaging. *PhD Thesis,* 2016